# EUROPEAN PATENT APPLICATION

(11) **EP 4 059 476 A1**
(43) Date of publication of application: **21.09.2022**
(21) Application number: 20905188.7
(22) Date of filing: 28.12.2020
(51) Int. Cl.: A61F 2/08, A61F 2/40

(54) **ROTATOR CUFF BALLOON**

(30) Priority: 27.12.2019 CN 201911380034
(71) Applicant: Shanghai Endophix Co., Ltd., Shanghai 201203 (CN)
(72) Inventor: YUE, Bin, Shanghai 201203 (CN); LIU, Chen, Shanghai 201203 (CN)
(74) Representative: Marks & Clerk LLP
(86) International application number: PCT/CN2020/139993
(87) International publication number: WO 2021/129860

(57) **Abstract**

A rotator cuff balloon (10) includes a limiting structure (100) and a protective structure (200) connected to the limiting structure (100). The limiting structure (100) has a curvature along a coronal plane (102). This rotator cuff balloon (10) conforms to the physiological structure of the shoulder joint in the human body, limits itself in the subacromial space and can reduce a patient's foreign body sensation, dislocation, functional failure and other adverse events. The protective structure (200) is configured to be supported in the space between the humeral head and the acromion of the shoulder joint in the human body to provide support. Moreover, the humeral head of a patient with a rotator cuff injury is raised, avoiding pain arising from inter-tissue collisions, increasing the moment arm of the deltoid muscle and resulting in immediate improvements in the functions of the patient's shoulder joint. The limiting structure (100) is configured to fit against at least part of the humeral head of the shoulder joint in the human body, thus providing a position-limiting effect and avoiding displacement of the prosthesis.

## Description

### TECHNICAL FIELD

The present application relates to the field of medical devices and, in particular, to rotator cuff balloon.

### BACKGROUND

The rotator cuff is a musculotendinous structure connecting the scapula to the head of the humerus and is located lateral to the glenohumeral joint capsule and medial to the deltoid muscle. The rotator cuff consists of the anterior rotator cuff (subscapularis muscle), the superior rotator cuff (supraspinatus muscle) and the posterior rotator cuff (infraspinatus and teres minor muscles). In addition to the functions of enabling a certain extent of internal rotation, external rotation and abduction of the arm, a more primary function of the rotator cuff is to stabilize the position of the humeral head on the glenoid, avoiding the humeral head from moving upward into collision with the acromion, which may lead to pain or the like. Therefore, the rotator cuff plays a crucial role in maintaining stability of the shoulder joint and in movements of the shoulder joint. However, with the advancement of age, subacromial bone proliferation may develop due to long-term repeated shoulder joint movements, or repeated strenuous movements may lead to wear and tear of the subacromial soft tissue (the bursa of the joint, the rotator cuff), which may impair the stability and movements of the humeral head, making the shoulder joint unable to move to permit the patient to abduct, elevate or otherwise move his/her arm. Moreover, the patient's quality of life and self-care ability will be significantly degraded because he or she may not be able to fall asleep due to severe pain arising from collisions between bones and between bones and the rotator cuff.

At present, therapies for rotator cuff injuries primarily include, among others, surgery and prosthesis implantation. Although good outcomes can be expected from surgical treatment of mild rotator cuff injuries, for injuries larger than 3 cm, surgical treatment would be uncertain in efficacy and suffer from easy recurrence. Prosthesis implantation artificially restricts upward movement of the humeral head to avoid pain resulting from inter-tissue collisions and increase the moment arm of the deltoid muscle, resulting in immediate improvements in the functions of the patient's shoulder joint. In the prior art, prosthetic implants such as prosthetic devices, biologic spacers or the like may be displaced during patients' activities. For example, the displacement of a prosthetic device may cause changes in pressure distribution, which may lead to rupture of the prosthesis and release of the contained material that may have deteriorated in quality due to a long-term stay at 37 °C, causing further damage to the affected part. Moreover, its filling hole is made of a hard material which, during a particular movement after the implantation, may cause discomfort and a foreign body sensation. Displacement of a biologic spacer may cause patient discomfort, limit his/her activities, or even damage the surrounding soft tissue, causing additional injury. Further, it is likely for prosthesis implants in the prior art to experience failure.

### SUMMARY

In view of this, there is a need to provide a rotator cuff balloon which conforms to the physiological structure of the human shoulder joint and limits itself in the subacromial space, thus solving the implant displacement problem and reducing the occurrence of adverse events.

In order to achieve the above goal, the present application discloses a rotator cuff balloon characterized in including: a balloon body including a limiting structure and a protective structure connected to the limiting structure, the limiting structure having a maximum width that is greater than a maximum width of the protective structure; a balloon interface disposed at an opening of the balloon body so as to allowing the passage of a filler material therethrough into the balloon body; a sealing member disposed on the balloon body and/or at the balloon interface, the sealing member including a sealing body, the sealing body including a sealing membrane for preventing the filler material from flowing out of the balloon body; and an outer catheter detachably connected to the balloon interface so as to allow the filler material to enter the balloon body from the outer catheter via the balloon interface.

Additionally, the protective structure may have a height H1 of 4 mm to 14 mm and the limiting structure may have a height H2 equal to HI + 15 mm.

Additionally, a height H2 of the limiting structure along a sagittal plane may be equal to HI + 15 mm.

Additionally, the protective structure may include curved line segments, wherein lines connecting two end points of the curved line segments and points defining the maximum width of the limiting structure define an isosceles trapezoid.

Additionally, the balloon body may include at least two layered structures both made of polyethylene.

Additionally, the balloon body may include an inner layer, an outer layer and a drug layer between the inner and the outer layers.

Additionally, the outer layer may be composed of a degradable polymer material, and/or the drug layer may contain at least one of the following drugs: diclofenac diethylamine, fentanyl and analogs thereof, etorphine and analogs thereof, the α2 receptor agonist medetomidine, droperidol, etomidate, vecuronium bromide and analogs thereof, procainamide hydrochloride, tetracaine hydrochloride, lidocaine hydrochloride, antibiotics and cephalosporin-based anti-inflammatory drugs.

Additionally, the present application discloses another rotator cuff balloon including a balloon body, a balloon interface and a sealing member, the balloon body including at least two layered structures both made of polyethylene, the balloon body including a limiting structure and a protective structure connected to the limiting structure, the limiting structure having a maximum width that is greater than a maximum width of the protective structure.

Additionally, the balloon interface may be disposed at an opening of the balloon body so as to allowing the passage of a filler material therethrough into the balloon body, wherein the sealing member is disposed on the balloon body and/or at the balloon interface and includes a sealing body, the sealing body including a sealing membrane for preventing the filler material from flowing out of the balloon body.

Additionally, the rotator cuff balloon may further include an outer catheter detachably connected to the balloon interface so as to allow the filler material to enter the balloon body from the outer catheter via the balloon interface.

Additionally, the sealing body may be disposed within the balloon body, wherein the sealing member includes an auxiliary tube detachably connected to the sealing body and configured to pre-position the sealing body at the opening of the balloon body.

Additionally, the protective structure may include curved line segments, wherein lines connecting two end points of the curved line segments and points defining the maximum width of the limiting structure define an isosceles trapezoid.

Additionally, the protective structure may have a height H1 of 4 mm to 14 mm and the limiting structure may have a height H2 equal to HI + 15 mm.

The rotator cuff balloon provided in the present application conforms to the physiological structure of the shoulder joint in the human body, limits itself in the subacromial space and can reduce a patient's foreign body sensations, dislocation, functional failure and other adverse events. The protective structure is adapted to be supported in the space between the humeral head and the acromion of the shoulder joint in the human body to provide support. Moreover, the humeral head of a patient with a rotator cuff injury is raised, avoiding pain arising from inter-tissue collisions, increasing the moment arm of the deltoid muscle and resulting in immediate improvements in the functions of the patient's shoulder joint. The limiting structure is adapted to fit against at least part of the humeral head of the shoulder joint in the human body, thus providing a position-limiting effect and avoiding displacement of the prosthesis.

In the rotator cuff balloon provided in the present application, the limiting structure has a first surface and a second surface, which are both curved so as to enable the limiting structure to fit against the entire humeral head or greater tubercle in the human body or part thereof. In this way, the limiting structure is enabled to provide a maximum position-limiting effect and maximally prevent displacement of the prosthesis.

In the rotator cuff balloon provided in the present application, an edge of the limiting structure is so curved to match the physiological shapes of the shoulder joint and the top of the rotator cuff, without limiting activities of the patient or damaging the surrounding soft tissue.

In the rotator cuff balloon provided in the present application, the limiting structure has a first accommodating chamber, and the protective structure has a second accommodating chamber in communication with the first accommodating chamber. The filler material is filled in the limiting and protective structures in such a manner that the protective structure can fit against the rotator cuff to avoid it from colliding with the acromion or other tissue structures during shoulder joint movements. Moreover, the humeral head and the acromion can be kept at a distance, maintaining such a moment arm length for shoulder joint movements that can reduce muscular loads. The limiting structure can tightly interlock with the humeral head of the shoulder joint in the human body and thus have improved position-limiting ability. In the limiting and protective structures, a liquid, a gel or a gas may be injected and filled to achieve their conformity to the physiological structure of the joint, resulting in desirable improvements in the shoulder joint functions of a patient with a rotator cuff injury. Further, as the special profile of the prosthesis matches the special physiological structure of the shoulder joint in the human body, it can provide better support, reduce collisions and achieve better therapeutic outcomes.

In order to reduce a patient's foreign body sensation that may follow the implantation of the rotator cuff balloon provided in the present application, when the filler material has been filled in the first and second accommodating chambers, a height of the limiting structure gradually increases from the end distal from the protective structure to the end where it is connected to the protective structure, i.e., the limiting structure is designed to be thin laterally and gradually thicken toward the proximal end. As such, the limiting structure will less affect the humeral head during movements.

The rotator cuff balloon provided in the present application allows the balloon body to be effectively sealed to prevent the filling liquid or gel from flowing out of the balloon and is simple in structure.

### BRIEF DESCRIPTION OF THE DRAWINGS

Fig. 1 a schematic diagram of various anatomical planes of the human body;
Fig. 2 is a schematic side cross-sectional view of a rotator cuff balloon in an expanded configuration according to an embodiment of the present application;
Fig. 3 is a schematic top view of the rotator cuff balloon of Fig. 1;
Fig. 4 is a schematic back view of the rotator cuff balloon of Fig. 1;
Fig. 5 is a schematic front view of the rotator cuff balloon of Fig. 1;
Fig. 6 is a schematic side view of the rotator cuff balloon of Fig. 1;
Fig. 7 is a schematic diagram showing the rotator cuff balloon of Fig. 1 that is being fitted in the human body;
Fig. 8A is a schematic side view of a rotator cuff balloon according to another embodiment, in which L1 denotes a length of the rotator cuff balloon along the coronal plane; L2, a length of a protective structure in the rotator cuff balloon along a coronal plane; HI, a height of the protective structure in the rotator cuff balloon along a sagittal plane; and H2, a height of the rotator cuff balloon along the sagittal plane;
Fig. 8B is a schematic top view of a rotator cuff balloon according to another embodiment, in which L3 denotes a width of a protective structure in the rotator cuff balloon along a horizontal plane, and L4 represents a width of a limiting structure in the rotator cuff balloon along the horizontal plane;
Fig. 9 is a schematic top view of a rotator cuff balloon according to another embodiment;
Fig. 10 is a schematic top view of a rotator cuff balloon according to another embodiment; and
Fig. 11 is a schematic diagram showing a sealing structure for a rotator cuff balloon according to another embodiment.

In these figures,
10: rotator cuff balloon; 100: limiting structure; 110: first accommodating chamber; 200: protective structure; 210: second accommodating chamber; 300: filling hole; 400: filling tube; 500: one-way valve; 600: filler material.

### DETAILED DESCRIPTION

In order to facilitate understanding of the present application, below, reference is made to related accompanying drawings to more fully describe the application. In the accompanying drawings, preferred embodiments of this application are presented. However, this application can be embodied in many different forms and are not limited to the embodiments set forth herein. Rather, the purpose of providing these embodiments is enable a more thorough and comprehensive understanding of the disclosure of the application.

Unless otherwise defined, all technical and scientific terms used herein have the same meaning as commonly understood by one having ordinary skill in the art to which the present application belongs. The terminology used in the specification of this application is for the purpose of describing particular embodiments only and is not intended to be limiting of the application. As used herein, the term "and/or" includes any and all combinations of one or more of the associated listed items.

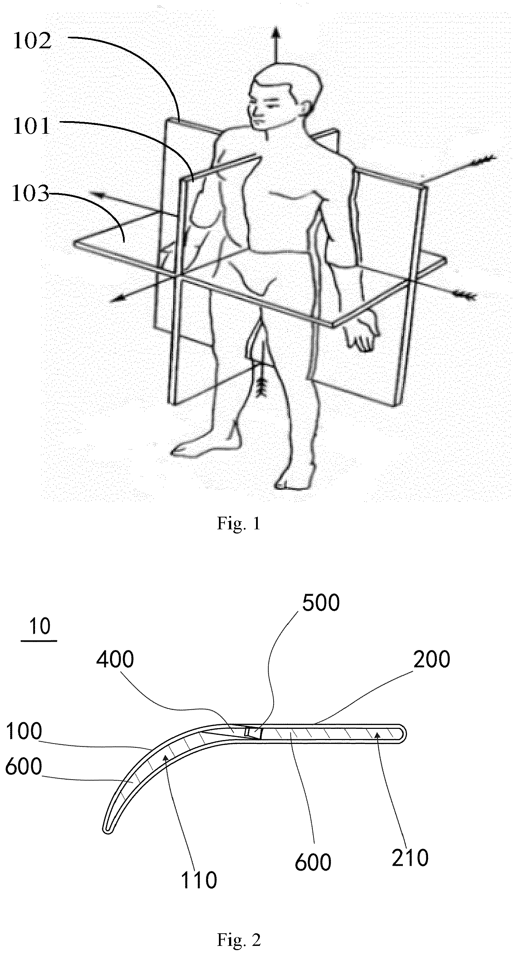

Reference is made to Fig. 1, a schematic diagram defining various anatomical planes of the human body: a sagittal plane 101, which is a longitudinal plane dividing, along the anteroposterior direction, the human body or a joint into left and right portions, wherein a sagittal plane passing through the middle of the human body is a median sagittal plane that divides the human body into equal left and right portions; a coronal plane 102, which is a longitudinal plane dividing, along the left-right direction, the human body or a joint into anterior and posterior portions and is perpendicular to the sagittal plane; a horizontal plane 103, also called a transverse plane, which is a plane parallel to the ground, dividing the human body or a joint into superior and inferior portions and perpendicular to both the coronal and sagittal planes.

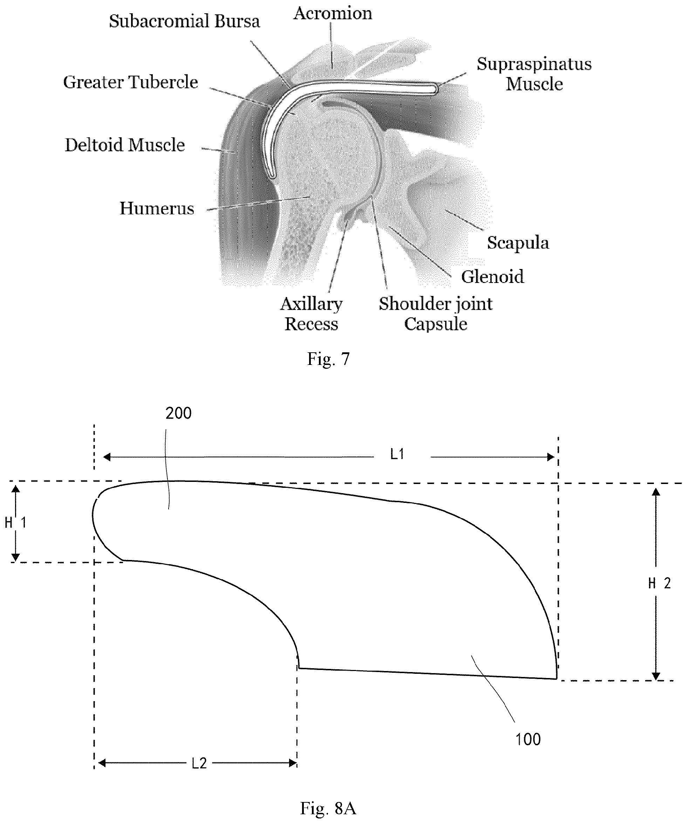

Referring to Fig. 2, one embodiment of the present application provides a rotator cuff balloon 10 including a limiting structure 100 and a protective structure 200 connected to the limiting structure 100. The limiting structure 100 has a curvature along the coronal plane. Alternatively, the limiting structure 100 forms an angle with the protective structure 200 along the coronal plane. Preferentially, the limiting structure 100 has a curvature along both the coronal and sagittal planes. The limiting structure 100 has an inner surface with a radius of curvature of 10 to 50 mm. Specifically, the limiting structure 100 has a first surface and a second surface. The first and second surfaces of the limiting structure 100 are both curved surfaces. For example, the second surface is the inner surface (as shown in Fig. 2, the surface facing down). Referring to Fig. 7, the second surface is adapted to fit against the entire humeral head and/or greater tubercle or part thereof. This second surface extends from the humeral head so as to cover at least part of the greater tubercle. The inventors have found that, compared with only covering the humeral head, covering the entire greater tubercle or part thereof with the second surface of the limiting structure 100 can prevent displacement or dislocation of the prosthesis during abduction, internal rotation or another movement of the arm, and can protect a torn wound of a rotator cuff tendon on a medial side of the prosthesis' limiting structure, further avoiding severe pain that the patient would experience when the torn wound collides with a bone. Therefore, the limiting structure 100 is enabled to provide an optimal position-limiting effect and maximally prevent displacement of the rotator cuff balloon. Preferably, the limiting structure 100 may be a half-bowl-shaped structure. Such a half-bowl-shaped limiting structure 100 can fit against the humeral head and function to maintain the position of the entire rotator cuff balloon. It can be understood that, in other embodiments, the structure and shape of the limiting structure 100 are not limited to those as described above, and may be a half-basin-shaped structure, a half-alms-bowl-shaped structure, or the like.

Referring to Fig. 7, the protective structure 200 is adapted to be supported in the space between the humeral head and the acromion of the shoulder joint in the human body to support the acromion.

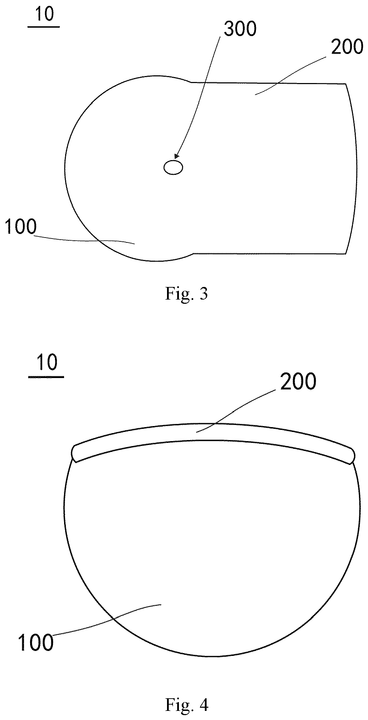

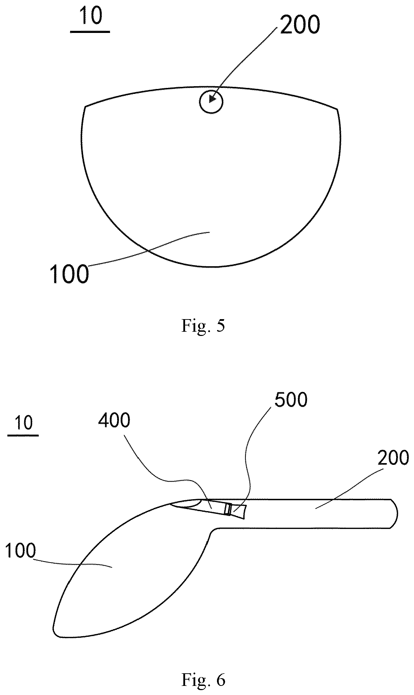

Additionally, referring to Figs. 4 and 5, an edge of the limiting structure 100 is curved along the sagittal plane. The edge of the limiting structure 100 is smooth. This curved structure can match the physiological shapes of the shoulder joint and the top of the rotator cuff, reducing damage caused by the lateral edge of the limiting structure 100 to muscular tissue during movements of the shoulder joint.

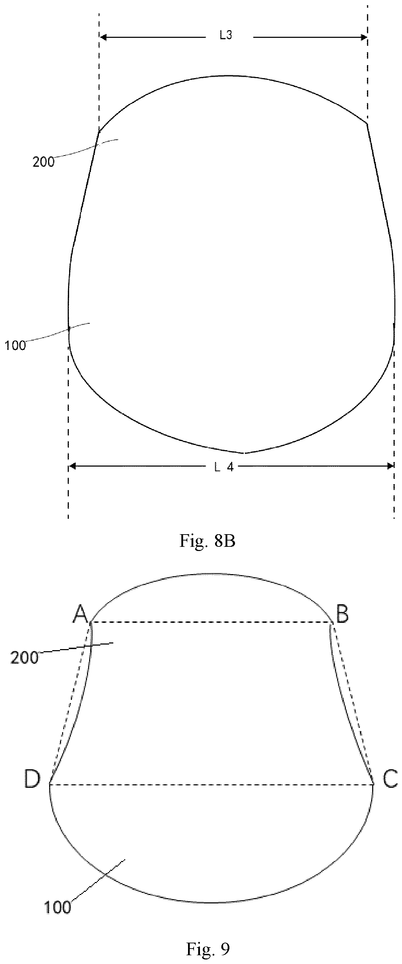

Additionally, referring to Figs. 2 and 3, a maximum width of the limiting structure 100 is greater than a maximum width of the protective structure 200. Preferably, referring to Fig. 8B, a width L4 of a transition between the limiting structure 100 and the protective structure 200 is greater than a width L3 of the protective structure 200. This arrangement can increase the conformity of the limiting structure 100 to the humeral head and the greater tubercle of the shoulder joint in the human body and cooperate with the fold of the subacromial bursal plica to limit displacement of the protective structure 200 and the entire rotator cuff balloon. Preferably, the limiting structure 100 and/or the protective structure 200 can deform, and the deformation may be recoverable compressive deformation, expansive deformation resulting from the filling of a filler material, or another form of deformation. The limiting structure 100 and/or protective structure 200 may deform so as to adapt to the humeral head of the shoulder joint in the human body and the space between the humeral head and the acromion of the shoulder joint in the human body and match the special physiological structure of the shoulder joint in the human body, providing better support, accomplishing of reducing collisions and achieving better therapeutic outcomes. During the filling, the limiting structure 100 and/or protective structure 200 can adapt itself/themselves, by deformation such as self-expansion after being compressed or expansion due to being filled, to the physiological structure of the shoulder joint, fit against the humeral head in the human body, and cooperate with the physiological structure of the fold of the bursal plica in the shoulder joint to limit medial or lateral displacement of the rotator cuff balloon in the shoulder joint, thus limiting itself/themselves in the subacromial space without dislocation. Further, the deformation-based self-adaptability enables the limiting structure 100 and the protective structure 200 to neither limit the patient's activities nor damage the surrounding soft tissue.

In another embodiment, referring to Fig. 8A, HI, a dimension of the protective structure 200 in the rotator cuff balloon 10 along the sagittal plane, i.e., a height of the protective structure 200, is preferred to be 4-14 mm. According to the principles of fluid mechanics of a closed chamber under pressure, *P*=*σ·*h / r, where P is a maximum pressure that the chamber can withstand, *σ* is the hoop stress intensity of the chamber housing material, h is a wall thickness of the chamber housing, and r is a minimum radius of the chamber. In the subacromial space where the shoulder joint prosthesis of the present application is to be implanted, a maximum squeezing pressure between the acromion and the humeral head is about 88 KPa (i.e., P=88KPa), and the height HI of the protective structure 200 in the shoulder joint prosthesis is 4-14 mm (i.e., the minimum radius of the protective structure's chamber r = 2-7 mm). Thus, the value of *σ·*h is in the range of 176-616 KPa·mm. This value is of significance in guiding the choice of a material for the shoulder joint prosthesis and in designing a particular wall thickness. For example, when the wall thickness h of the shoulder joint prosthesis is 0.1 mm, it is applicable to at least choose a material whose intensity *σ* is in the range of 1760-6160 KPa for the prosthesis. In fact, commonly used non-degradable and degradable medical materials such as polyamide, polyester, polyethylene, polylactic acid and polycaprolactone can meet this requirement. Therefore, a preferred HI value of 4-14 mm can impart to the protective structure the ability to support the humeral head and the acromion when the shoulder joint prosthesis is fabricated from a common used medical material with conventional fabrication accuracy.

H2, a height of the rotator cuff balloon 10 along the sagittal plane, is preferred to be 9-34 mm. More preferably, a straight-line length of the limiting structure 100 along the sagittal plane, i.e., a height H2 of the limiting structure 100 is equal to HI + 15 mm. This arrangement enables the limiting structure 100 to cover the humeral head and the greater tubercle while avoiding the limiting structure 100 from impeding movements of the rotator cuff muscle group due to oversize.

Referring to Fig. 8A, L1, a length of the rotator cuff balloon 10 along the coronal plane is preferred to be 30-70 mm. L2, a length of the protective structure 200 in the rotator cuff balloon 10 along the coronal plane is preferred to be 10-40 mm. Referring to Fig. 8B, L3, a width of the protective structure 200 in the rotator cuff balloon 10 along the horizontal plane is preferred to be 15-60 mm. L4, a width of the limiting structure 100 in the rotator cuff balloon 10 along the horizontal plane is preferred to be 20-70 mm. This arrangement enables the protective structure 200 to separate the acromion from the humeral head and allows the limiting structure 100 to cover the humeral head and the greater tubercle to prevent dislocation.

Different sizes of the rotator cuff balloon 10 are suitable for different humeral head diameters. For example, for a humeral head diameter of 5 cm, preferably, HI is 10-12 mm, H2 is 25-27 mm, L1 is 55-65 mm, L2 is 25-35 mm, L3 is 30-50 mm, L4 is 55-60 mm and the radius of curvature of the inner surface of the limiting structure 100 is 25-30 mm.

Referring to Figs. 8A to 8B, the height H2 of the limiting structure 100 gradually increases from the end distal from the protective structure 200 to the end where it is connected to the protective structure 200.

Fig. 9 is a top view of another embodiment of the shoulder joint prosthesis. As shown in Fig. 9, the maximum width of the limiting structure 100 is defined between points D and C, and the protective structure 200 includes curved line segments ending respectively at points A and B. The points A, B, C, D defines an isosceles trapezoid. This structure can enhance horizontal force transmission between line segments AB and CD of the protective structure. When the humeral head rotates toward the medial side of the glenoid fossa during abduction and elevation of the arm, a horizontal force directed from CD to AB is exerted on and horizontally compresses the protective structure. This trapezoidal contour design with large taper angles can reduce loss of the force during its transmission between the aforesaid two boundary line segments and thus corrugated deformation caused by internal strain of the protective structure during the dynamic process of arm abduction. More preferably, the curved line segment AB has a curvature ≤0.21 mm⁻¹, and the curved line segment CD has a curvature ≤0.15 mm⁻¹.

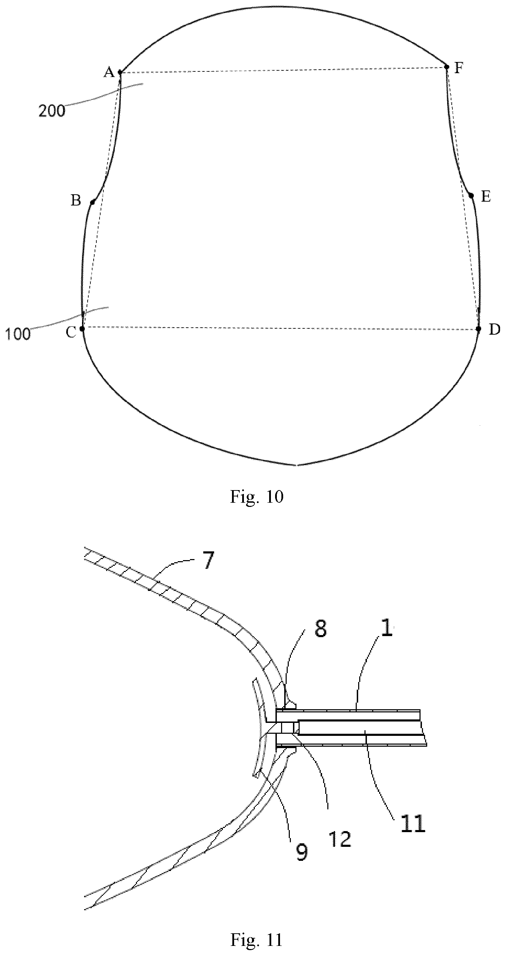

In Fig. 9, points A and D may be connected by one or more straight line segments, or by one or more curved line segments, or by both straight line segment(s) and curved line segment(s). As shown in Fig. 10, points A and C are connected by a curved segment and a curved segment BC, and points D and F are connected by a curved segment DE and a curved segment EF. Each of the curved line segments AB and EF preferably has a curvature ≤0.3mm⁻¹, and each of the curved line segments BC and DE preferably has a curvature ≤0.15 mm⁻¹.

The limiting structure 100 has a first accommodating chamber 110, and the protective structure 200 has a second accommodating chamber 210 in communication with the first accommodating chamber 110. Both the first accommodating chamber 110 and the second accommodating chamber 210 are both adapted to be filled by a filler material 600. After the limiting structure 100 and the protective structure 200 are filled with the filler material 600 during the filling process, the protective structure 200 can fit against the rotator cuff, avoiding the rotator cuff from colliding with the acromion or other tissue structures during shoulder joint movements. Moreover, the humeral head and the acromion can be kept at a distance, maintaining a moment arm length for shoulder joint movements, which can reduce muscular loads. The limiting structure 100 can tightly mesh with the humeral head of the shoulder joint in the human body and thus have improved position-limiting ability.

Preferably, the limiting structure 100 and the protective structure 200 may be balloons, or sponge-like components, or non-expandable structures.

When the limiting structure 100 has the first accommodating chamber 110 and the protective structure 200 has the second accommodating chamber 210, preferably, a maximum height of the limiting structure 100 gradually increases from the end distal from the protective structure 200 to the end where it is connected to the protective structure 200. A minimum height of the limiting structure 100 is its height before the filler material is filled or after the limiting structure 100 is compressed. The maximum height of the limiting structure 100 is its height after the filler material is filled or after it has recovered by itself from the compressed configuration. A minimum height of the protective structure 200 is its height before the filler material is filled or after the protective structure 200 is compressed. A maximum height of the protective structure 200 is its height after the filler material is filled or after it has recovered by itself from the compressed configuration.

Referring to Fig. 2, when the first accommodating chamber 110 and the second accommodating chamber 210 are both being filled with the filler material 600, the height of the limiting structure 100 gradually increases from the end distal from the protective structure 200 to the end where it is connected to the protective structure 200. In order for the patient to have a reduced foreign body sensation after the rotator cuff balloon 10 is implanted, the height of the limiting structure 100 is configured to gradually increase from the end distal from the protective structure 200 to the end where it is connected to the protective structure 200, i.e., the limiting structure 100 is designed to be thin laterally and gradually thicken toward the proximal end, when the first accommodating chamber 110 and the second accommodating chamber 210 are both being filled with the filler material 600. As such, the limiting structure 100 will less affect the humeral head during movements.

In addition, a chamber wall of the protective structure 200 may be specifically optimized for different particular different application needs. For example, for those in need of more motion activities, it may be appropriately thickened for more years of service.

Additionally, when the first accommodating chamber 110 and the second accommodating chamber 210 are both being filled with the filler material 600, the height of the limiting structure 100 at the end where it is connected to the protective structure 200 may equal to the height of the protective structure 200, in order to reduce discomfort of the humeral head during shoulder joint movements.

One surface of the protective structure 200 is so curved as to substantially fit against the rotator cuff. Specifically, the protective structure 200 is a structure curved downward with respect to the transverse plane. This curved structure matches the physiological shapes of the shoulder joint and the top of the rotator cuff, and after the filler material 600 is filled, the protective structure 200 can fit against the rotator cuff and prevent the rotator cuff from colliding with the acromion or other tissue structures during movements of the shoulder joint. Further, the humeral head and the acromion can be kept at a distance, maintaining a moment arm length for shoulder joint movements, which can reduce muscular loads.

Further, referring to Figs. 3 and 5, the limiting structure 100 and/or the protective structure 200 is provided with a filling hole 300, the filling hole 300 is in communication with both the first accommodating chamber 110 and the second accommodating chamber 210. The filling hole 300 is provided therein with a one-way valve 500 which can avoid reflowing of the filler material 600 that has been filled via the filling hole 300.

Additionally, referring to Figs. 2 and 6, the above rotator cuff balloon 10 further includes a filling tube 400 which is disposed within the first accommodating chamber 110 and/or the second accommodating chamber 210 and brought into communication with the filling hole 300. An opening of the filling tube 400 facing the outside is flush with an outer surface of the limiting structure 100 or the protective structure 200, and the one-way valve 500 is received in the filling tube 400.

Further, referring to Fig. 6, a diameter of the filling tube 400 at the end where it is brought into communication with the filling hole 300 is smaller than a diameter thereof at the end facing the inside.

Preferably, the filling tube 400 is flexible, effectively solving the prior art problem of discomfort and a foreign body sensation of the patient that may be caused by particular movements after the implantation.

In addition, referring to Fig. 6, the limiting structure 100 is connected to the protective structure 200 to form an integral structure. Designing the limiting structure 100 and the protective structure 200 as such an integral structure facilitates moldmaking and fabrication at low fabrication cost.

Further, the limiting structure 100, the protective structure 200 and the filling tube 400 are all made of a non-degradable material. According to the present application, fabricating both the limiting structure 100 and the protective structure 200 in the rotator cuff balloon 10 from a non-degradable material solves the prior art problem that implants may fail to degrade within short terms, avoids rupture of the rotator cuff balloon 10, enables it to effectively operate for a long time at a temperature of 37 °C, and effectively avoids the filler material that has deteriorated in quality from being released and causing further damage to the affected part.

Additionally, materials from which the limiting structure 100 can be fabricated include, but are not limited to, one or more of silicone, polyurethane, rubber, polyamide, polyester and polyolefin. Materials from which the protective structure 200 can be fabricated include, but are not limited to, one or more of silicone, polyurethane, rubber, polyamide, polyester and polyolefin. Materials from which the filling tube 400 can be fabricated include, but are not limited to, silicone, polyurethane, rubber, polyamide, polyester and polyolefin. In a preferred embodiment, in order to enable the rotator cuff balloon to provide long-term support for the acromion, the balloon is preferably implemented as a double-layer balloon, and both layers of the double-layer balloon are made of polyethylene. The inventors have found that, the double-layer polyethylene rotator cuff balloon has greatly enhanced puncture resistance and can achieve self-adaptability by effectively filling a joint cavity in the human body when inflated.

Further, referring to Fig. 2, the above rotator cuff balloon 10 further includes the filler material 600 that is filled in the first accommodating chamber 110 and the second accommodating chamber 210. Preferably, the filler material 600 includes a liquid and/or a gel. For example, the filler material 600 is water, silicone, a gel or the like.

Fig. 11 is a schematic cross-sectional view of a sealing member for the rotator cuff balloon disclosed in the present application. The sealing member includes a sealing body 9 disposed within a balloon body 7 and an auxiliary tube 11 detachably connected to the sealing body 9. By means of the auxiliary tube 11, the sealing body 9 may be positioned to cover a balloon interface 8. Before a filling process begins, the auxiliary tube 11 is pre-connected to the sealing body 9, and an outer catheter 1 is then connected to the balloon interface 8. The filling process begins with opening a chamber in the balloon body 7 by pushing the auxiliary tube 11 and hence the sealing body 9. The balloon body 7 is then filled using an annular gap between the outer catheter 1 and the auxiliary tube 11. After the filling process finishes, the sealing body 9 is fitted against the opening of the balloon body 7 under pressure of the filler material therein. At this point, the outer catheter 1 and the auxiliary tube 11 are removed and withdrawn from the human body, with the balloon body 7 remaining sealed. The auxiliary tube 11 or the sealing body 9 may be designed with a weakened area 12. When an operator applies a pulling force to the auxiliary tube 11, the sealing body 9 is fitted against the opening of the balloon body 7 under internal pressure therein, and the weakened area 12 is broken. In alternative embodiments, mating threads may be provided on one end of the sealing body 9 and on the auxiliary tube 11, or one end of the sealing body 9 may be engaged with the auxiliary tube 11 by an interference fit.

In a preferred embodiment, a drug may be loaded on a surface of or inside the rotator cuff balloon provided in the present application.

In a preferred embodiment, the drug-loaded rotator cuff balloon is capable of immediate of the drug. After the rotator cuff balloon is implanted into the human body and expanded therein, an outer surface of the balloon is brought into contact with tissue and fluids in the human body, leading to immediate release of the drug on the outer surface of the balloon. A drug-eluting coating may be loaded on the outer surface of the balloon. Additionally, grooves may be pre-formed in the outer surface of the balloon, in order for an increased amount of the drug to be loaded. On the balloon surface, a drug capable of facilitating the recovery of injured tendons, an analgesic drug, an anti-inflammatory drug or another drug may be loaded in order to provide an effect of facilitating the growth and recovery of torn tendons, alleviating the patient's pain, eliminating inflammations or the like.

The balloon may be chosen as a drug-eluting balloon or structured with drug-loading grooves in an outer surface of the chamber wall.

The drug loaded on the balloon may be chosen as one or a combination of diclofenac diethylamine, fentanyl and analogs thereof, etorphine and analogs thereof, the α2 receptor agonist medetomidine, droperidol, etomidate, vecuronium bromide and analogs thereof, procainamide hydrochloride, tetracaine hydrochloride, lidocaine hydrochloride, antibiotics, cephalosporin-based anti-inflammatory drugs and other drugs capable of facilitating tendon recovery or providing an analgesic or anti-inflammatory effect.

The balloon may be inflated and expanded by a gas, a liquid or a gel. The inflating medium may be determined as required by a physician.

The balloon-inflating substance may be chosen as a drug solution or gel, and the balloon material may be chosen as a biodegradable polymer material for medical use. After the balloon is degraded, the drug contained therein may be released to the lesion site.

In a preferred embodiment, the drug-loaded rotator cuff balloon is capable of timed drug release. This rotator cuff balloon is a double-layer, double-chamber balloon structure having an outer chamber for drug storage and an inner chamber for enabling inflation and expansion of the balloon. The outer balloon layer is chosen as a biodegradable polymer material for medical use. When the outer chamber wall is degraded, the drug contained in the outer chamber of the double-layer double-chamber structure balloon will come into contact with tissue and fluids in the human body, achieving release of the drug and allowing it to provide a therapeutic effect. Quantitative degradation time regulation for the outer balloon layer can be achieved by adjusting a composition, chain and block structures, molecular weight and degree of crystallinity of the polymer as the outer balloon layer material and a thickness of the chamber wall. Upon the elapse of a clinically required time after the balloon is implanted, the outer chamber wall of the balloon will be completely degraded, allowing the drug to be released in a time manner. The inner balloon layer may be made either of a degradable biopolymer material for medical use or of a non-degradable polymer material for medical use.

The balloon may be expanded by inflating the inner chamber with a gas, a liquid or a gel. The inflating medium may be determined as required by a physician. When the inner balloon layer is chosen as a biodegradable polymer material for medical use, the inner chamber may also be inflated using a drug solution or gel.

An outer surface of the balloon's outer chamber wall may not be loaded with a drug, or employ the structure of the above-described drug-load balloon for immediate drug release. The drug may be eluted from the outer surface of the outer chamber wall, or loaded grooves formed in the chamber wall outer layer.

In a preferred embodiment, the drug-loaded rotator cuff balloon is capable of periodic drug release. On the basis of the structure of the above drug-loaded balloon for timed release, a drug-loaded multilayer balloon structure having multiple chambers is provided, of which, the innermost one serves as an inflation chamber and all the remaining ones are drug-loaded chambers. Each of the drug-loaded chambers has an outer layer made of a biodegradable polymer material for medical use. When this chamber wall of the drug-loaded chamber is degraded, a drug in the drug-loaded chamber will come into contact with tissue and fluids in the human body, achieving release of the drug. Since the outermost chamber wall of the balloon first comes into contact with fluids in the human body, the chamber walls of the drug-loaded chambers are successively degraded from the outside inward. In this way, the drug in the multiple drug-loaded chambers will periodically come into contact with tissue and fluids in the human body as a result of the degradation of the respective outer layers, achieving release of the drug. Depending on therapeutic requirements, either a single drug or multiple drugs of different types and for different uses may be loaded in the individual drug-loaded chambers. Quantitative degradation time regulation for the outer layers of the drug-loaded chambers can be achieved by adjusting compositions, chain and block structures, molecular weights and degrees of crystallinity of the polymers as the outer layer materials of the drug-loaded chambers and thicknesses of the chamber walls. The innermost balloon layer (i.e., the outer layer of the inflation chamber) may be made either of a degradable biopolymer material for medical use or of a non-degradable polymer material for medical use.

In the multilayer, multi-chamber balloon, the innermost chamber is adapted to allow inflation and expansion of the balloon, and the remaining chambers are all adapted for drug loading. The multiple chamber walls may be fused together by hot melt welding at a tube section of the balloon or in the vicinity thereof, sealing the individual drug-loaded chambers. When the innermost balloon layer is made of a non-degradable material, the multiple chamber walls may be connected together using glue, thus sealing the drug-loaded chambers.

In the balloon, the outer layers of the drug-loaded chambers are each made of a biodegradable polymer material for medical use, while the innermost layer may be made either of a non-degradable material or of a biodegradable polymer material for medical use.

In a preferred embodiment, the drug-loaded rotator cuff balloon is capable of long-term sustained drug release. This balloon may appear like a non-drug-loaded balloon and employ a single-chamber structure. After implanted into the human body, a drug solution or a drug gel may be injected into the chamber to inflate and expand the balloon. The balloon chamber wall is formed of a porous polymer membrane, and driven by a concentration difference of the drug's components between inside and outside the balloon as well as by pressure from bones and tissue in the human body, the drug will be released out of the balloon at a certain rate in a sustained manner through pore channels. Depending on the molecule size of the used drug, the porous material for the balloon may be pre-formed to contain compatibly sized micropores (with a pore size <2 nm) or mesopores (with a pore size of 2-50 nm). Moreover, quantitative regulation of the drug release rate and time can be achieved by adjusting the porosity, pore size, tortuosity factor, thickness and other structural characteristic parameters of the porous membrane for balloon chamber wall. This balloon structure is advantageous over the other structures in a greater drug load and a longer drug release time. The present application further provides a sponge-like porous drug-loaded implant structure which is saturated with a drug solution that is absorbed and retained in the sponge-like structure. After the structure is implanted into the human body, the drug solution will be released under pressure from bones and tissue in the human body. This sponge-like implant may be combined with a balloon structure to form a composite structure with overall enhanced support performance. In this implant, the balloon is configured as an inner member, and the sponge-like porous drug-loaded structure as an outer member.

The sponge-like porous structure is made of a biocompatible elastic polymer material for medical use, which may be selected from rubber-based thermosetting elastomers and thermoplastic elastomers such as polyamide, polyurethane, polyolefin and polystyrene. In the composite structure, a material for the balloon is selected as a biocompatible polymer for medical use, which may be selected from polyesters, polyamide, polyvinyl chloride, nylon elastomers, polyurethane and so on.

The various technical features of the foregoing embodiments may be combined in any way. Although not all such combinations have been described above for the sake of brevity, any of them is considered to fall within the scope of this specification as long as there is no contradiction between the technical features.

Presented above are merely several embodiments of the present application. Although these embodiments are described with some particularity and in some detail, it should not be construed that they limit the scope of the present application in any sense. It should be note that various variations and modifications can be made by those of ordinary skill in the art without departing from the concept of the present application. Accordingly, it is intended that all such variations and modifications are embraced within the scope of this application as defined in the appended claims.

## Claims

1. A rotator cuff balloon, comprising:
a balloon body comprising a limiting structure and a protective structure connected to the limiting structure, the limiting structure having a maximum width that is greater than a maximum width of the protective structure;
a balloon interface disposed at an opening of the balloon body, the balloon interface configured to allow the passage of a filler material therethrough into the balloon body;
a sealing member disposed on the balloon body and/or at the balloon interface, the sealing member comprising a sealing body, the sealing body comprising a sealing membrane for preventing the filler material from flowing out of the balloon body; and
an outer catheter detachably connected to the balloon interface so as to allow the filler material to enter the balloon body from the outer catheter via the balloon interface.

2. The rotator cuff balloon of claim 1, wherein the protective structure has a height HI of 4 mm to 14 mm and the limiting structure has a height H2 equal to HI + 15 mm.

3. The rotator cuff balloon of claim 1, wherein the protective structure comprises curved line segments, and wherein lines connecting two end points of the curved line segments and points defining the maximum width of the limiting structure define an isosceles trapezoid.

4. The rotator cuff balloon of claim 1, wherein the balloon body comprises at least two layered structures both made of polyethylene.

5. The rotator cuff balloon of claim 4, wherein the balloon body comprises an inner layer, an outer layer and a drug layer between the inner layer and the outer layer.

6. The rotator cuff balloon of claim 5, wherein the outer layer is composed of a degradable polymer material, and/or the drug layer comprises at least one of the following drugs: diclofenac diethylamine, fentanyl and analogs thereof, etorphine and analogs thereof, the α2 receptor agonist medetomidine, droperidol, etomidate, vecuronium bromide and analogs thereof, procainamide hydrochloride, tetracaine hydrochloride, lidocaine hydrochloride, antibiotics and cephalosporin-based anti-inflammatory drugs.

7. A rotator cuff balloon, comprising a balloon body, a balloon interface and a sealing member, the balloon body comprising at least two layered structures both made of polyethylene, the balloon body comprising a limiting structure and a protective structure connected to the limiting structure, the limiting structure having a maximum width that is greater than a maximum width of the protective structure.

8. The rotator cuff balloon of claim 7, wherein the balloon interface is disposed at an opening of the balloon body, the balloon interface configured to allow the passage of a filler material therethrough into the balloon body, and wherein the sealing member is disposed on the balloon body and/or at the balloon interface and comprises a sealing body, the sealing body comprising a sealing membrane for preventing the filler material from flowing out of the balloon body.

9. The rotator cuff balloon of claim 8, further comprising an outer catheter detachably connected to the balloon interface so as to allow the filler material to enter the balloon body from the outer catheter via the balloon interface.

10. The rotator cuff balloon of claim 7, wherein the sealing body is disposed within the balloon body, and wherein the sealing member comprises an auxiliary tube detachably connected to the sealing body and configured to pre-position the sealing body at the opening of the balloon body.

11. The rotator cuff balloon of claim 7, wherein the protective structure comprises curved line segments, and wherein lines connecting two end points of the curved line segments and points defining the maximum width of the limiting structure define an isosceles trapezoid.

12. The rotator cuff balloon of claim 7, wherein the protective structure has a height HI of 4 mm to 14 mm and the limiting structure has a height H2 equal to HI + 15 mm.
